Europäisches Patentamt

European Patent Office

Office européen des brevets

(11) Veröffentlichungsnummer : **0 116 944**
**B1**

# (12) EUROPÄISCHE PATENTSCHRIFT

(45) Veröffentlichungstag der Patentschrift :
20.08.86

(21) Anmeldenummer : 84101527.4

(22) Anmeldetag : 15.02.84

(51) Int. Cl.⁴ : **A 61 K 31/21, C 07 C 77/02**

(54) Glycerinmononitrate zur Behandlung der Angina pectoris.

(30) Priorität : 18.02.83 DE 3305690

(43) Veröffentlichungstag der Anmeldung :
29.08.84 Patentblatt 84/35

(45) Bekanntmachung des Hinweises auf die Patenterteilung : 20.08.86 Patentblatt 86/34

(84) Benannte Vertragsstaaten :
AT BE CH DE FR GB IT LI LU NL SE

(56) Entgegenhaltungen :
CHEMICAL ABSTRACTS, Band 71, Nr. 3, 21. Juli 1969,
Seite 271, Nr. 12540e, Columbus, Ohio, USA

(73) Patentinhaber : Heinrich Mack Nachf.
Postfach 140
D-7918 Illertissen (DE)

(72) Erfinder : Leitold, Matyas, Dr.
Klauflügelweg 21
D-7950 Biberach (DE)
Erfinder : Stoss, Peter, Dr. Dipl.-Chem.
Mozartstrasse 15
D-7918 Illertissen (DE)

(74) Vertreter : Lederer, Franz, Dr. et al
Patentanwälte Dr. Lederer Franz Meyer-Roxlau Reiner F. Lucile-Grahn-Strasse 22
D-8000 München 80 (DE)

EP 0 116 944 B1

## Beschreibung

Glycerin α-mononitrat ist einerseits ein Sprengstoff, andrerseits besitztes gefäßerweiternde Eigenschaften (Chem. Abstr. Vol. 71, 1969, ref. 12 570 e).

Verschiedene Forscher haben gezeigt, daß das pharmakokinetische Verhalten von Glycerintrinitrat (GTN) in Ratten, Hunden und Menschen nicht mit der pharmakodynamischen Wirkung übereinstimmt. Die kurzen, intravenösen Plasma-Eliminierungs-Halbwertzeiten für GTN zwischen 2,8 und 12,2 min in verschiedenen Tierarten und Menschen und die länger anhaltende intravenöse oder enterale Wirksamkeit zeigen klar, daß sich der pharmakodynamische Einfluß nicht durch GTN alleine ergibt. Diese Tatsache gab den Anlaß, den pharmakologischen und pharmakokinetischen Einfluß von Glycerin-1-nitrat (1-GN) und Glycerin-2-nitrat (2-GN) zu untersuchen.

Die Mononitrate, 1-GN und 2-GN, sind bekanntlich Metaboliten von GTN, die auftreten, sobald GTN einmal die Leber passiert. Manche Autoren haben berichtet, daß GTN als Hypotensivum nach oraler Verabreichung inaktiv ist, und andere haben berichtet, daß die Mononitrate inaktiv sind, wenn sie intravenös verabreicht wurden. Enterale Verabreichung der Mononitrate von GTN würden daher erwartungsgemäß pharmakologisch nicht wirksam sein.

Überraschenderweise wurde nun gefunden, daß Glycerin-1-nitrat und/oder Glycerin-2-nitrat für die Herstellung von pharmazeutischen Zusammensetzungen für orale, intravenöse oder sublinguale Verabreichung, enthaltend als aktiven Bestandteil etwa 5 bis 90 Gew.-%, bezogen auf das Gewicht der gesamten Zusammensetzung, Glycerin-1-nitrat und/oder Glycerin-2-nitrat in einem pharmazeutisch annehmbaren Träger, bei der Behandlung von Angina pectoris und pulmonaler Hypertension brauchbar sind.

Bevorzugt sind der aktive Bestandteil Glycerin-1-nitrat oral in einer Tagesmenge von etwa 60-400 mg oder intravenös oder sublingual zu 20-130 mg verabreicht und der aktive Bestandteil Glycerin-2-nitrat oral in einer Tagesmenge von etwa 240-2 400 mg oder intravenös oder sublingual zu 80-800 mg verabreicht.

In dieser Anmeldung hat der Begriff « orale Verabreichung » seine übliche Bedeutung ; d. h., daß das zu verabreichende Material geschluckt wird. Der Begriff « Behandlung », angewandt in Verbindung mit Angina pectoris und pulmonaler Hypertension, umfaßt sowohl die Behandlung von Symptomen als auch die Verhinderung des Zustands. Aus dem Nachfolgenden wird hervorgehen, daß der primäre Vorteil der Mononitrate die Wirkungsdauer des Wirkstoffs ist.

Der Einfluß von GTN und 1-GN auf narkotisierte Tiere (Ratten, Hunde und Kaninchen) nach intravenöser oder intraduodenaler Applikation ist in den Tabellen 1 und 2 zusammengefaßt. Die Stärke des hypotensiven Effekts der beiden Verbindungen war nach i. v.-Applikation klar voneinander verschieden, in Abhängigkeit von der angewandten Konzentration ; kurze Wirkung von GTN bei geringer Konzentration, lange Wirkung mit hoher Dosierung von 1-GN. Intraduodenal bei Hunden und Kaninchen appliziert war 1-GN nur geringfügig schwächer als GTN. In Ratten war es 38 mal schwächer in den Wirkung als GTN. Die Wirkungsdauer von 1-GN ging deutlich über die von GTN bei allen Tierarten hinaus.

Enteral ruft GTN im Vergleich zur i. v.-Applikation eine viel länger dauernde Wirkung hervor.

Sowohl GTN als auch 1-GN senkten des systolischen Blutdruck für unterschiedliche Zeitdauer nach sublingualer Applikation bei Hunden, wie in Tabelle 3 gezeigt. Der Beginn der Wirkung war am schnellsten mit GTN (0,4 min) gefolgt von 1-GN (2,1 min). 1-GN war in höherer Konzentration für viel längere Zeiten wirksam als GTN.

Nach intraduodenaler Verabreichung an narkotisierte Ratten setzten alle drei Substanzen dosisabhängig die durch Vasopressin induzierten Koronarspasmen herab, Tabelle 4. Die prophylaktisch orale Applikation der Substanzen zu verschiedenen Zeiten bei der gleichen Tierart rief gegenüber Vasopressin eine 2-3 h dauernde antianginöse Wirkung hervor, Tabelle 5.

Die pharmakokinetischen Vergleichsuntersuchungen (i. v., p. o.) mit 1-GN an einem wachen Hund zeigen eine Bioverfügbarkeit der Substanz von 58 % an. Die Halbwertzeit für 1-GN wurde nach 3,4 h bestimmt. Diese Halbwertzeit für 1-GN in Hunden korreliert mit der pharmakodynamisch hypotensiven Aktivität des Wirkstoffs.

Eine Eliminierungs-Halbwertzeit von 2,1 h wurde für 2-GN nach intravenöser Applikation an einen wachen Hund bestimmt. Diese Zeit korreliert mäßig mit der pharmakodynamischen Wirkung von 16 mg/kg p. o. 2-GN oder 32 mg/kg p. o. bei einem wachen Hund.

Beim Testen der antianginös wirksamen Substanz gehört die Messung der Wirksamkeit bei gesunden Tieren in die erste Phase. Für diesen Zweck eignet sich die Methode des Hervorrufens von Koronarspasmen in narkotisierten Ratten durch Vasopressin am besten. Die Hypoxie in der Koronararterie wird durch eine ST-Wellensenkung oder im frühen Stadium durch eine T-Wellenerhöhung ausgedrückt. Alle drei Nitrate hemmten die T-Wellenerhöhung im EKG von Ratten nach intraduodenaler Applikation, dosisabhängig, wodurch GTN entschieden den stärksten Einfluß hatte (Tabelle 4). Eine Korrelation besteht zwischen der hypotensiven Wirkung von 1-GN und der antianginösen Wirksamkeit in Ratten nach i. d.-Applikation, da beide Wirkungen bei der gleichen Konzentration beobachtet wurden. Andere Autoren untersuchten die Wirkung von GTN, Isosorbid-dinitrat (ISDN), Verapamil, Nifedipin und die von Dipyramidol in Ratten hinsichtlich der ST-Wellensenkung nach intravenöser, sublingualer oder oraler Applikation. Beide organischen Nitrate hemmten die ST-Wellensenkung intravenös, Nitroglycerin hemmte auch nach sublingualer Applikation, ISDN und die beiden Calcium-Antagonisten hemmten nach oraler Applikation, Dipyridamol war unwirksam. In unseren Versuchen beeinflußten GTN, 1-GN und 2-GN die Vasopressin-induzierten Koronarspasmen für 2-3 h in einer oralen Dosierung von 40 mg/Tier. Die Wirkungsdauer von GTN entspricht nicht der Halbwertzeit in der Ratte, 21-30 min. Im Gegensatz erreichte der maximale GN-Wert in Rattenserum seinen höchsten Wert nach 1,5 h und wurde mit einer Halbwertzeit von 2,6 h eliminiert.

Innerhalb 5 min nach intraduodenaler Applikation von Nitroglycerin an Ratten konnte kein weiterer Plasmawert der Ausgangssubstanz gemessen werden. Bei intraduodenaler Applikation von GTN an narkotisierte Ratten konnte eine hypotensive Wirkung von 14 min bei einer dreimal höheren Dosierung nachgewiesen werden. Die längere antianginöse Wirkung von Nitroglycerin in Ratten kann deshalb nur der Ausgangssubstanz in der ersten Phase, bis zu 1 h, zugeschrieben werden, danach muß sie ihren rasch gebildeten Metaboliten zugeschrieben werden. Dies wird auch durch die kurze orale Halbwertzeit der Substanz in Ratten und die sehr hohen für eine antianginöse Wirkung erforderlichen oralen Konzentrationen angezeigt. Die hypotensive Wirkung von GTN wurde durch Vorbehandlung der Tiere mit intravenösem Dihydroergotamin (DHE) herabgesetzt. Die hypotensive Wirkung von 1-GN wurde nach Vorbehandlung mit DHE erheblich herabgesetzt.

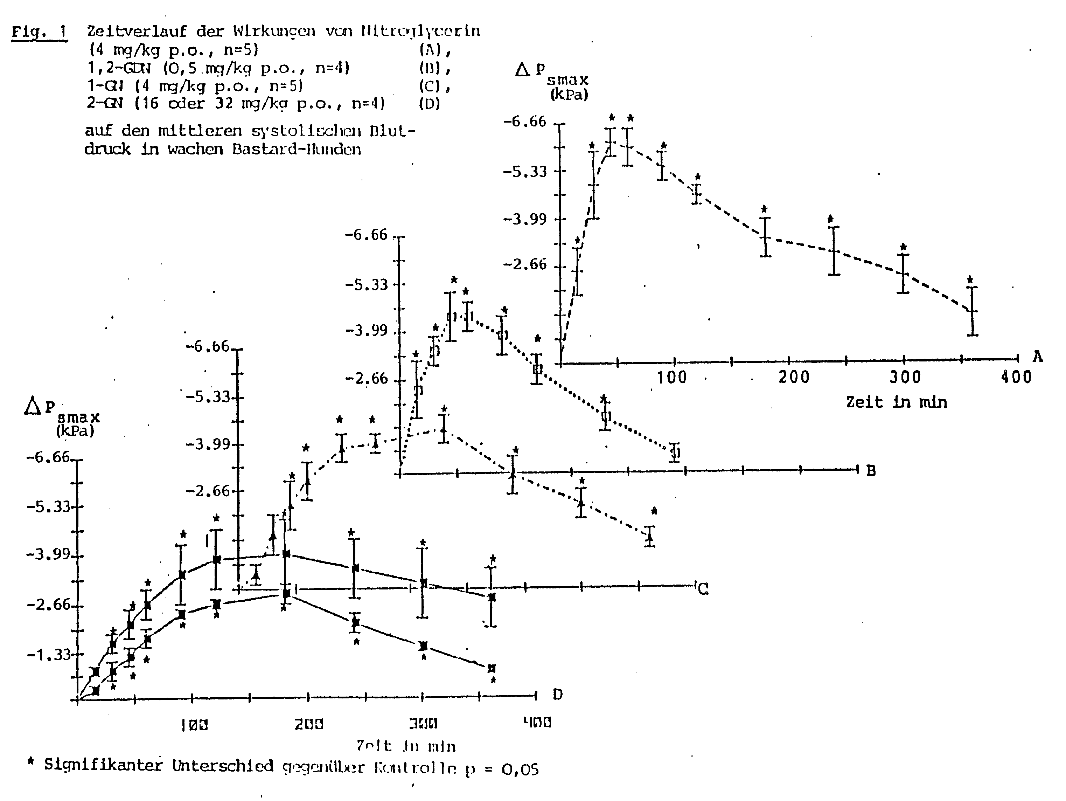

Die in den Tabellen 1-6 und Fig. 1 gezeigten Ergebnisse wurden wie folgt erhalten :
Wirkung auf den Blutdruck anästhesierter Ratten, Kaninchen und Hunde.

Kaninchen beiderlei Geschlechts (Gewicht 2,5-3,5 kg) oder männliche Sprague-Dawley-Ratten (Gewicht 350-450 g) oder Bastard-Hunde (Gewicht 18-25 kg) wurden mit Natriumpentobarbiton (60 mg/kg i. v. oder 40 mg/kg i. p.) anästhesiert. Eine Femoralarterie wurde zur Messung des Blutdrucks mit einem Druckaufnehmer (Statham, ModellP 23 Db), verbunden mit einem Polygraph (Modell Schwarzer, München) mit einer Kanüle versehen. Die EKG-Führung wurde mit Hilfe von Nadel-Elektroden über einen Eka-Impuls durchgeführt, was eine gleichzeitige Aufzeichnung der Herzfrequenz ermöglicht. Die Atmung wurde mit Hilfe eines Pneumotachographen aufgezeichnet. Zur Verabreichung von Wirkstoffen als Suspension in 1 % CMC wurden Kanülen in den Zwölffingerdarm der Tiere gebracht. Die Mittelwerte wurden für systolischen und diastolischen Blutdruck vor und nach Verabreichung der Wirkstoffe (n = 5-6) errechnet.

Wirkung auf den Blutdruck bei wachen Hunden.

Bastard-Hunde (Gewicht 18-25 kg) wurden bei den Untersuchungen verwendet. Der Blutdruck wurde direkt bei wachen Tieren mit Hilfe eines in die Aorta ascendens über die A. circumflexa ilea eingeführten Verweilkatheters, verbunden mit einem Statham-Druckaufnehmer, gemessen. Die Wirkstoffe wurden entweder als stetige intravenöse Infusion durch Steigerung der Wirkstoff-Dosierungswerte alle 20 min, Aufhören der Infusion und Messen des systolischen Blutdrucks ohne Wirkstoffe für weitere 20 min oder sublingual oder oral in Gelatinekapseln verabreicht. Die Mittel wurden für den systolischen Blutdruck berechnet, und die $ED_{20}$-Werte (mmHg, i. e.) wurden durch Varianz oder Kovarianz-Analyse mit Vertrauensgrenzen (n = 4-5) bestimmt, das sind die Dosen, die einen Abfall des systolischen Blutdrucks von 20 mmHg verursachten.

Antianginöse Aktivität in Ratten

Die Methode beruht auf dem Wirkstoff-induzierten Abklingen von ischämischen EGK-Änderungen, gekennzeichnet durch Bradykardie und verlängerte (5-20 s) T-Wellenerhöhung, ausgelöst durch 0,5 IE/kg Vasopressin i. v. in mit Pentobarbital anästhesierten männlichen Ratten (150-200 g). 10 Tiere wurden für jede Dosis, jedes Mal und Verbindung eingesetzt. Kontrolltiere wurden oral mit 1 % Methylcellulose (CMC) dosiert. Die Wirkstoffe wurden p. o. 1-4 h vor der i. v. Vasopressin-Verabreichung verabreicht. Die Ergebnisse wurden als Prozent Hemmung der Vasopressin-induzierten Koronarspasmen gegenüber Kontrolltieren ausgedrückt. In einem weiteren Versuch wurden die antianginösen Wirkstoffe i. d. 2 min vor der i. v. Vasopressin-Verabreichung verabreicht. Bei der Datenauswertung zeigte sich Linearität der Dosis-Reaktion-Beziehung durch Varianz- und Kovarianzanalyse mit Vertrauensgrenzen für p = 0,05.

Orale Dosen von 1-GN werden wahrscheinlich unverändert in den systemischen Kreislauf ohne Stoffwechsel bei der ersten Leberpassage aufgenommen. Diese pharmakologische Wirkung war die gleiche nach i. v. oder oraler Verabreichung bei den drei verschiedenen getesteten Tierarten. Wirkstoffe, die einen Stoffwechsel bei der ersten Passage erfahren (GTN), müssen in höheren oralen Dosen gegeben werden und unterliegen breiten Schwankungen im Stoffwechsel von einem Subjekt zum anderen.

Needleman und Mitarbeiter (1972) haben geltend gemacht, daß es keine rationale Basis für die orale Verabreichung lang wirkender Nitrate bei der Behandlung von Angina pectoris gibt. Diese Aussage beruht auf ihrer Beobachtung, daß anästhesierte Ratten nicht auf intraportale Injektion von Nitraten (ISDN = Isosorbiddinitrat GTN) reagierten und daß diese Wirkstoffe nur sehr niedrige und kurze Serum-Konzentrationen, aber hohe Konzentrationen ihrer Stoffwechselprodukte (ISMNs, GDNs, GMNs) hervorriefen. In den letzten Jahren wurden starke Hinweise dafür geboten, daß ISDN und seine Mononitrat-Metaboliten, oral verabreicht, oder GTN und seine Dinitrat-Metaboliten, sublingual oder oral verabreicht, hämodynamisch und antianginös in verschiedenen Versuchsmodellen wirksam sind. (Leitold et al., Krankenhausarzt 56, 112 (1983) ; Leitold et al., Iuphar 9th International Congress of Pharmacol., 29 July - 03 August 1984, p. 47).

Die intravenöse Wirksamkeit der beiden GTN-Dinitrat-Metaboliten wurde bereits 1968 und 1969 von zwei Autoren beschrieben sowie die Unwirksamkeit von den GTN-Mononitraten 1969.

Bogaert, MG., MT. Rosseel, AF. Schaepdryver : Arch. int. Pharmacodyn 176, 458 (1968).

Needleman, P., DJ. Blehm, KS. Rotskoff : J. Pharmacol. Exper. Ther. 165, 268 (1969).

In einer neuen Übersichtsarbeit über Nitroglycerin wird ausdrücklich die vasodepressorische Wirkung der GTN-Dinitrat Metaboliten bestätigt oder zitiert, die der Mononitrate aber verneint. (Curry, SH., SM. Aburawi : Biopharm. Drug Dispos. 6, 235, 249 (1985)).

Die folgenden Beispiele dienen der Veranschaulichung

## Beispiel 1

Glycerin-1-nitrat, 20 mg-Tabletten

| Zusammensetzung | mg/Tablette |
|---|---|
| (1) Glycerin-1-nitrat-Lactose-Pulver, 20 % | 100,0 |
| (2) Lactose | 200,0 |
| (3) Mikrokristalline Cellulose | 174,0 |
| (4) Magnesiumstearat | 4,0 |
| (5) Talcum | 1,6 |
| | 480,0 |

(1), (2), (3), (4) und (5) werden gesiebt, ausreichend und sorgfältig gemischt und auf einer geeigneten Tablettenpresse gepreßt.

## Beispiel 2

Glycerin-1-nitrat, 20 mg-Kapseln

| Zusammensetzung | mg/Kapsel |
|---|---|
| (1) Glycerin-1-nitrat-Lactose-Pulver, 20 % | 100,0 |
| (2) Lactose | 47,5 |
| (3) Mikrokristalline Cellulose | 80,0 |
| (4) Magnesiumstearat | 2,5 |
| | 230,0 |

(1), (2), (3) und (4) werden gesiebt, ausreichend und sorgfältig gemischt und in Hartgelatinekapseln, Größe 3, auf einer geeigneten Kapselfüllmaschine gefüllt.

## Beispiel 3

Glycerin-1-nitrat-Tropfen

| Zusammensetzung | g/10 g |
|---|---|
| (1) Glycerin-1-nitrat-Sorbit-Pulver, 20 % | 2,0 |
| (2) p-Methyl-hydroxybenzoat | 0,01 |
| (3) Gereinigtes Wasser | 7,99 |
| | 10,0 |

(2) wird heiß in (3) gelöst. Dann wird hierin (1) gelöst. Die Lösung wird filtriert und in Tropfenfläschchen gefüllt.

## Beispiel 4

Glycerin-2-nitrat-60 mg-Tabletten

| Zusammensetzung | mg/Tablette |
|---|---|
| (1) Glycerin-2-nitrat-Lactose-Pulver, 20 % | 300,0 |
| (2) Mikrokristalline Cellulose | 174,4 |
| (3) Magnesiumstearat | 4,0 |
| (4) Talcum | 1,6 |
| | 480,0 |

(1), (2), (3) und (4) werden gesiebt, ausreichend und sorgfältig gemischt, und auf einer geeigneten Tablettenpresse zu Tabletten gepreßt.

Beispiel 5

Glycerin-2-nitrat-40 mg-Sublingual-Tabletten

| Zusammensetzung | mg/Tablette |
|---|---|
| (1) Glycerin-2-nitrat-Sorbit-Pulver, 20 % | 200,0 |
| (2) Natrium-stearylfumarat | 10,0 |
| | 210,0 |

(1) wird gesiebt, in einem geeigneten Granulator granuliert und in einer Trockenkammer auf Trögen getrocknet. Das trockene Granulat wird ausreichend und sorgfältig mit (2) gemischt und auf einer geeigneten Tablettenpresse zu Tabletten gepreßt.

Beispiel 6

Glycerin-2-nitrat-Tropfen

| Zusammensetzung | g/10 g |
|---|---|
| (1) Glycerin-2-nitrat-Sorbit-Pulver, 20 % | 2,0 |
| (2) p-Methyl-hydroxybenzoat | 0,01 |
| (3) Gereinigtes Wasser | 7,99 |
| | 10,0 |

(2) wird heiß in (3) gelöst. Dann wird (1) hierin gelöst. Die Lösung wird filtriert und in Tropffläschchen gefüllt.

Beispiel 7

Glycerin-1-nitrat-20 mg-Sublingual-Tabletten

| Zusammensetzung | mg/Tablette |
|---|---|
| (1) Glycerin-1-nitrat-Sorbit-Pulver, 20 % | 100,0 |
| (2) Natrium-stearylfumarat | 5,0 |
| | 105,0 |

(1) wird gesiebt, in einem geeigneten Granulator granuliert und in einer Trockenkammer auf Trögen getrocknet. Das trockene Granulat wird genügend und sorgfältig mit (2) gemischt und auf einer geeigneten Tablettenpresse zu Tabletten gepreßt.

Beispiel 8

Glycerin-1-nitrat-Injektionslösung

| Zusammensetzung | | mg/ml |
|---|---|---|
| (1) Glycerin-1-nitrat-Sorbit-Pulver, 20 % | | 50,0 |
| (2) Benzylalkohol | | 10,0 |
| (3) Wasser zur Injektion | zu | 1,0 ml |

(1) und (2) werden in (3) gelöst. Die Lösung wird durch Filtrieren sterilisiert und anschließend in Ampullen gefüllt.

Beispiel 9

Glycerin-1-nitrat-Suppositorien

| Zusammensetzung | mg/Supp. |
|---|---|
| (1) Glycerin-1-nitrat-Sorbit-Pulver, 20 % | 150,0 |

(2) Propylgallat 0,2
(3) Hartes Fett 1 849,8
2 000,0

(2) wird in der Schmelze (3) gelöst. (1) wird in dieser Schmelze suspendiert, und wenn nötig wird die Suspension durch eine Kolloidmühle geführt. Die Suspension wird mit einer geeigneten Suppositorienmaschine in vorgeformte Kunststoff-Suppositorien-Behälter gegossen, gekühlt und in den Behältern versiegelt.

(Siehe Tabellen Seite 7 ff.)

Tabelle 1

Einfluß von GTN und 1-GN auf den arteriellen Mitteldruck in mit Pentobarbiton anästhesierten Ratten

| Verbindung | n | Art der Verabrei-chung | Dosis | Arterieller Mitteldruck (kPa) $\bar{x}$ $s\bar{x}$ | maximale Wirkung (min) $\bar{x}$ $s\bar{x}$ | Wirkungsdauer (min) $\bar{x}$ $s\bar{x}$ |
|---|---|---|---|---|---|---|
| Kontrolle | 6 | i.v. | phys.NaCl-Lsg. | 13.06 ± 0.58 | | |
| GTN | 6 | i.v. | ′8.0 µg/kg | 8.13 ± 0.57* | 0.21 ± 0.02 | 1.9 ± 0.2 |
| Kontrolle | 6 | i.d. | phys.NaCl-Lsg. | 11.73 ± 0.42 | | |
| GTN | 6 | i.d. | 0.8 mg/kg | 8.39 ± 0.65* | 2.95 ± 0.09 | 13.7 ± 0.8 |
| Kontrolle | 6 | i.v. | phys.NaCl-Lsg. | 11.59 ± 0.73 | | |
| 1-GN | 6 | i.v. | 30.0 mg/kg | 9.19 ± 0.46* | 17.5 ± 0.7 | 76.8 ± 5.5 |
| Kontrolle | 6 | i.d. | phys.NaCl-Lsg. | 12.66 ± 0.73 | | |
| 1-GN | 6 | i.d. | 30.0 mg/kg | 9.59 ± 0.66* | 20.7 ± 0.8 | 73.7 ± 2.4 |

* Signifikanter Unterschied gegenüber Kontrolle p = 0.05

Tabelle 2

Einfluß von GTN und 1-GN auf den arteriellen Mitteldruck in narkotisierten Kaninchen und Hunden
(Pentobarbiton)

| Verbindung | Art | n | Art der Verab-reichung | Dosis | arterieller Mitteldruck (kPa) $\bar{x}$  $s\bar{x}$ | maximale Wirkung (min) $\bar{x}$  $s\bar{x}$ | Wirkungs-dauer (min) $\bar{x}$  $s\bar{x}$ |
|---|---|---|---|---|---|---|---|
| Kontrolle GTN | Kaninchen | 6 | i.v. | 8 µg/kg | 12.13 $\pm$ 0.22<br>8.93 $\pm$ 0.53* | 18 $\pm$ 5.0 | 5 $\pm$ 1 |
| Kontrolle GTN | Kaninchen | 6 | i.d. | 8 mg/kg | 10.66 $\pm$ 0.39<br>6.53 $\pm$ 0.66* | 4 $\pm$ 1 | >90 |
| Kontrolle 1-GN | Kaninchen | 6 | i.v. | 16 mg/kg | 10.39 $\pm$ 0.26<br>6.79 $\pm$ 0.66* | 7.0 $\pm$ 2 | >90 |
| Kontrolle 1-GN | Kaninchen | 6 | i.d. | 16 mg/kg | 12.39 $\pm$ 0.66<br>7.73 $\pm$ 0.39* | 18 $\pm$ 5 | >90 |
| Kontrolle GTN | Hund | 5 | i.v. | 8 µg/kg | 18.52 $\pm$ 1.59<br>11.86 $\pm$ 0.0* | 1 $\pm$ 0.1 | 16 $\pm$ 1 |
| Kontrolle GTN | Hund | 6 | i.d. | 8 mg/kg | 19.99 $\pm$ 1.46<br>14.79 $\pm$ 2.26* | 16 $\pm$ 3 | 49 $\pm$ 3 |
| Kontrolle 1-GN | Hund | 5 | i.v. | 16 mg/kg | 19.19 $\pm$ 1.33<br>14.92 $\pm$ 1.46 | 16 $\pm$ 3 | 103 $\pm$ 9 |
| Kontrolle 1-GN | Hund | 5 | i.d. | 16 mg/kg | 17.19 $\pm$ 0.79<br>11.46 $\pm$ 1.06 | 19 $\pm$ 3 | 130 $\pm$ 5 |

* Signifikanter Unterschied gegenüber Kontrolle p = 0,01

## Tabelle 3

Einfluß von Nitroglycerin, 0.5 mg/Hund, und 1-GN, 2 mg/kg sublingual, auf den systolischen Blutdruck bei wachen Bastard-Hunden. n = 3/Verbindung

| Verbindung | Dosis mg/kg oder mg/Hund | mittlerer systolischer Blutdruck (kPa) | maximale Wirkung (min) | Wirkungsdauer (min) |
|---|---|---|---|---|
| Kontrolle | 0.5/Hund | $18.26 \pm 1.06$ | $2.7 \pm 0.7$ | $16 \pm 0.9$ |
| GTN | | $15.06 + 0.66*$ $-3.06$ | | |
| Kontrolle | 2.0 mg/kg | $19.59 \pm 0.39$ | $25 \pm 2$ | 120 |
| 1-GN 1) | | $16.39 + 0.53*$ $-3.20$ | | |

* Signifikanter Unterschied gegenüber Kontrolle p = 0.05 1) 36 mg/Hund

0 116 944

## Tabelle 4

Wirkung verschiedener Verbindungen auf die T-Wellenerhöhung, ausgelöst durch Vasopressin i. v., im EKG der narkotisierten Ratte. Die Substanzen wurden i. d. 2 min vor Vasopressin i. v. verabreicht.

| Verbindung | n | Applikations art | Dosierung mg/kg | Abnahme der T-Wellen-erhöhung, $ED_{50}$ mg/kg $ED_{50}$ mg/kg | Vertrauensgrenzen p = 0.05 mg/kg |
|---|---|---|---|---|---|
| GTN | 24 | i.d. | 0.050 - 0.100 | 0.047 | 0.038 - 0.059 |
| '1-GN' | 24 | i.d. | 18.7 - 50.0 | 30.87 | 25.23 - 37.78 |
| 2-GN | 24 | i.d. | 25.0 - 100.0 | 68.72 | 45.52 - 103.73 |

0 116 944

## Tabelle 5

Prozent Hemmung der T-Wellenerhöhung, ausgelöst durch i. v. Vasopressin im EKG der narkotisierten Ratte. Die Verbindungen wurden prophylaktisch oral zu verschiedenen Zeiten verabreicht.

| Substanz | n/ Zeit | Applika-tionsart | Dosierung, mg/Tier | % Hemmung auf die T-Wellenerhöhung gegenüber Kontrollen nach | | | |
|---|---|---|---|---|---|---|---|
| | | | | 60 | 120 | 180 | 240 min |
| GTN | 8 | p.o. | 40 | −33★ | −39★ | −30★ | −17 |
| 1−GN | 8 | p.o. | 40 | −50★ | −32★ | −14 | + 2 |
| 2−GN | 8 | p.o. | 40 | −50★ | −32★ | −26★ | −20 |

* Signifikanter Unterschied gegenüber Kontrolle bei p = 0,05

Tabelle 6

Einfluß von GTN, 1-GN und 2-GN auf den systolischen Blutdruck in wachen Hunden. Die Wirkstoffe wurden als kontinuierliche intravenöse Infusion unter Erhöhung der Wirkstoff-Dosierungswerte alle 20 min. verabreicht. Beenden von Infusion und Messen des systolischen Blutdrucks für die folgenden 20 min. ohne Wirkstoffe.

| Verbindung | n | Dosis $\mu g/kg$ | systolischer Blutdruck (kPa) $\bar{x}$ $s\bar{x}$ | $\Delta P_s$ max (kPa) | $ED_{20}$ mmHg und Vertrauensgrenzen ($\mu g/kg$) p=0.05 |
|---|---|---|---|---|---|
| Kontrolle | 5 | phys.NaCl-Lsg. | 23.72 + 1.26 | --- | |
| | 5 | 2.0 | 22.26 + 1.30 | -1.46 | |
| | 5 | 4.0 | 21.19 + 1.42 | -2.53 | 4.51 (3.45 - 5.89) |
| | 5 | 8.0 | 20.26 + 1.18 | -3.46 | |
| ohne Wirkstoff- ende der Infusion | | | | | |
| 5 | 5 | | 21.72 + 1.23 | -1.99 | |
| 10 | 5 | | 22.39 + 1.19 | -1.33 | |
| 15 | 5 | | 22.79 + 1.19 | -0.93 | |
| 20 min | 5 | | 23.46 + 1.19 | -0.26 | |
| Kontrolle | 5 | phys.NaCl-Lsg. | 25.06 + 0.58 | --- | |
| 1-GN | 5 | 1000 | 23.46 + 0.82 | -1.59 | |
| | 5 | 2000 | 21.72 + 0.94 | -3.33 | 1.628 (1.327 - 1.998) |
| | 5 | 4000 | 20.39 + 0.70 | -4.66 | |
| ohne Wirkstoff- ende der Infusion | 5 | | 20.39 + 0.57 | -4.66 | |
| Kontrolle | 5 | phys.NaCl-Lsg. | 24.66 + 0.85 | --- | |
| 2-GN | 5 | 4000 | 22.79 + 0.71 | -1.86 | |
| | 5 | 8000 | 21.32 + 0.62 | -3.19 | 6.223 (5.144 - 7.528) |
| | 5 | 16000 | 20.39 + 0.55 | -4.26 | |
| ohne Wirkstoff- ende der Infusion | 5 | | 20.79 + 0.53 | -3.86 | |

**Patentanspruch**

Verwendung von Glycerin-1-nitrat und/oder Glycerin-2-nitrat für die Herstellung von oral, intravenös oder sublingual zu verabreichenden Arzneimitteln für die Behandlung von Angina pectoris und pulmonaler Hypertension.

**Claim**

Use of glycerin-1-nitrate and/or glycerin-2-nitrate for the preparation of oral, intravenous or sublingual medicaments for the treatment of angina pectoris and pulmonary hypertension.

**Revendication**

Utilisation de 1-nitrate de glycérine et/ou de 2-nitrate de glycérine pour la fabrication de médicaments orales, intraveineux ou sublinguaux pour le traitement d'angine de poitrine et des hypertensions pulmonales.

Fig. 1 Zeitverlauf der Wirkungen von Nitroglycerin
(4 mg/kg p.o., n=5)                    (A),
1,2-GDN (0,5 mg/kg p.o., n=4)          (B),
1-GN (4 mg/kg p.o., n=5)               (C),
2-GN (16 oder 32 mg/kg p.o., n=4)      (D)

auf den mittleren systolischen Blutdruck in wachen Bastard-Hunden

* Signifikanter Unterschied gegenüber Kontrolle p = 0,05